# DEMANDE DE BREVET EUROPEEN

(11) **EP 1 964 591 A1**
(43) Date de publication de la demande: **03.09.2008**
(21) Numéro de dépôt: 07290246.3
(22) Date de dépôt: 27.02.2007
(51) Int. Cl.: A61N 5/10, A61L 31/06

(54) **Bolus pour radiothérapie, et procédé pour déterminer la forme d'un tel bolus**

(71) Demandeur: Institut Curie, 75005 Paris (FR)
(72) Inventeur: Campana, François, 94340 Joinville (FR); Kristner, Jean-Yves, 94120 Fontenay (FR); Lachet, Jérémy, 92240 Malakoff (FR); Fournier-Bidoz, Nathalie, 78720 La Celle les Bordes (FR)
(74) Mandataire: Pontet, Bernard

(57) **Abrégé**

L'invention concerne un bolus destiné à recevoir une irradiation lors d'une radiothérapie. Avantageusement, ce bolus est réalisé à partir d'un gel polyuréthane de densité égale à 1 et composé préférentiellement d'un mélange de polyol et de dix pourcents d'isocyanate. Le bolus est avantageusement réalisé à froid.

## Description

La présente invention se rapporte à un bolus destiné à recevoir une irradiation lors d'une radiothérapie. Elle trouve une application particulièrement intéressante mais non limitative dans la radiothérapie du sein.

Par bolus, on entend un matériau, si possible équivalent-tissu, placé au contact d'une région irradiée pour corriger des irrégularités de surface ou pour donner à la distribution de dose en profondeur une forme adaptée aux structures anatomiques à protéger ou à irradier.

D'une façon générale, la radiothérapie devient incontournable dans le traitement des cancers du sein, quel que soit le stade d'évolution de la tumeur et quel que soit le protocole thérapeutique ; elle limite le risque de récidives locales de 70%. Ainsi, la radiothérapie est indispensable en chirurgie radicale où la mammectomie associée au curage axillaire est suivie d'une irradiation de la paroi thoracique et des aires ganglionnaires lymphatiques de drainage. Elle devient essentielle avec le développement des traitements conservateurs, la chirurgie se limitant à une tumorectomie et à un curage axillaire. Autrement dit, la radiothérapie est indispensable dans toutes les formes de cancers du sein notamment, associé le plus souvent à la chirurgie et parfois à la chimiothérapie.
En France , 50% de traitement en radiothérapie après mastectomie utilisent l'irradiation par faisceau d'électrons.
Le traitement consiste à irradier un volume cible qui peut être :
- le sein ou la paroi thoracique,
- la région axillaire,
- la région sus-claviculaire,
- la chaîne mammaire interne.
Or, le problème lié à l'irradiation est la présence d'organes sains à proximité immédiate de la cible à traiter. On doit éviter l'irradiation de ces organes sains qui peuvent par exemple comprendre :
- le poumon,
- le coeur,
- le plexus brachial,
- la moelle épinière cervicale,
- le larynx, ou
- la thyroïde.

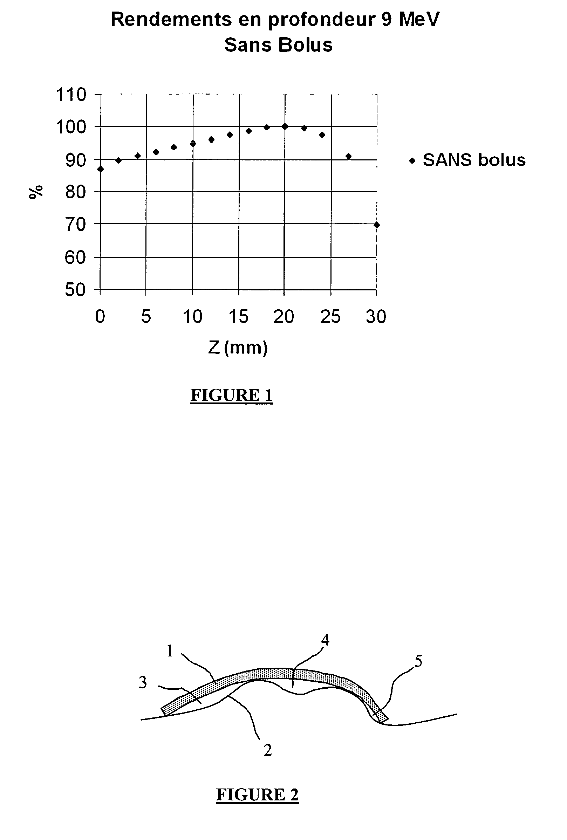

Sur la figure 1 selon l'art antérieur, on voit une courbe représentant le rendement énergétique d'une irradiation de faisceau d'électrons en fonction de la profondeur (Z) dans le corps. Des phénomènes physiques font que le rendement ne suit pas une décroissance linéaire depuis la peau jusqu'à l'intérieur du corps. Le rendement présente plutôt un maximum de dose à une certaine profondeur par rapport à la peau puis décroît rapidement de manière exponentielle. Dans la plupart des cas, il est nécessaire de décaler ce maximum pour que ce dernier se situe exactement sur la cible tout en épargnant au maximum les organes sains. Sur la figure 1 par exemple, le maximum de dose est à une profondeur de 20mm. Pour réaliser un décalage, la pratique courante consiste à placer un bolus sur la zone à traiter de façon à augmenter artificiellement l'épaisseur de la peau et réduire ainsi la profondeur de pénétration du faisceau d'irradiation. Le bolus le plus couramment utilisé est en silicone vendu par tranche d'une épaisseur allant de 5 à 10mm. Ce bolus en silicone est difficilement maniable et reste onéreux à l'achat. Sur la figure 2 selon l'art antérieur, on voit un tel bolus posé sur un corps dont la surface présente une forme irrégulière. A cause de la rigidité relative de la silicone, les espaces 3, 4 et 5 se forment entre le corps 2 et le bolus 1. Par ailleurs, on comprend aisément que la pénétration du faisceau d'étectrons ne soit pas homogène. Les inconvénients du bolus en silicone sont donc essentiellement :
- une maniabilité difficile,
- une densité différente de 1, ce bolus en silicone n'est pas équivalent tissu,
- un coût élevé, et
- une disponibilité par tranche d'épaisseur fixe, ce qui ne permet pas d'épouser convenablement la surface irrégulière d'un corps humain : ceci conduit à une inhomogénéité de la distribution de doses dans le volume cible.
On connaît le document US 6,231,858 qui décrit un bolus pour radiothérapie constitué d'un gel aqueux. Ce gel est élaboré à partir d'un mélange de polymères organiques naturels et d'eau. Ce bolus est peu coûteux et peut être conformé de façon à corriger la distribution de doses. Cependant, la fabrication de ce bolus nécessite de chauffer l'eau à une température entre 70 et 100°C avant d'y incorporer un polymère organique naturel. Ce mode de fabrication à haute température est très contraignant et nécessite un contrôle permanent et sévère afin d'éviter notamment la présence de bulles d'air.

Par conséquent, la présente invention a pour but de remédier au plus grand nombre des inconvénients précités en proposant une nouvelle composition de bolus pour radiothérapie.
La présente invention a en particulier pour but un nouveau bolus simple à réaliser et peu onéreux.
Un autre but de l'invention est un bolus facile à modeler et acceptant des découpages très fins et non simplement un empilement de couches.

On atteint au moins l'un des objectifs précités avec un bolus destiné à recevoir une irradiation lors d'une radiothérapie. Selon l'invention, ce bolus comprend du gel polyuréthane. Le bolus selon la présente invention peut être conçu de manière aisée. En effet le gel polyuréthane est un matériau provenant d'un mélange de deux composants à température ambiante et à pression normale. Le durcissement se fait à froid, donc pas de chauffage et pas de phénomène de contraction. L'utilisation d'un moule est donc aisée.
Au contraire, le bolus décrit dans le document US 6 231 858 nécessite une mise en oeuvre complexe notamment en chauffant à une température entre 70 et 100°.
Par ailleurs, le gel polyuréthane selon l'invention est un matériau peu onéreux largement moins coûteux que la silicone par exemple.

L'invention est notamment remarquable par le fait que le gel polyuréthane est un matériau très utilisé dans l'industrie du bâtiment, dans la réalisation de siège pour moto, dans les semelles de chaussure ou encore dans des équipements de santé pour absorber des chocs de faible intensité. Son utilisation massive en industrie mécanique explique notamment son faible coût.

Avantageusement, le gel polyuréthane selon l'invention présente une densité sensiblement égale à 1, ce qui évite tous problèmes liés à la transition bolus-corps. On obtient un bolus équivalent tissu.
Avantageusement, le bolus est constitué d'un gel polyuréthane composé d'un mélange de polyol et d'isocyanate.
Selon une caractéristique avantageuse de l'invention, le gel polyuréthane comprend au plus quinze pourcents d'isocyanate, de préférence entre cinq et quinze pourcents. Plus précisément, ce gel polyuréthane comprend sensiblement de huit à dix pourcents d'isocyanate et de quatre-vingt-douze à quatre-vingt-dix pourcents de polyol. Une telle répartition permet d'obtenir un gel polyuréthane facile à travailler avec une élasticité, une dureté et un aspect collant idéaux.
Selon une caractéristique avantageuse de l'invention, ce bolus est destiné à être plaqué sur le corps. Il présente une forme permettant de placer sensiblement le maximum de dose dans une cible prédéfinie au sein dudit corps. La texture du gel polyuréthane selon l'invention permet de réaliser des détails très fins. Il est facile à mouler et à découper.
Le bolus selon l'invention permet d'assurer une fonction de compensateur vis-à-vis de l'irradiation et permet d'avoir une irradiation homogène sur l'ensemble de la cible.
Selon un mode de réalisation préférentiel de l'invention, la forme du bolus est telle que le bolus épouse parfaitement la surface du corps et l'épaisseur de l'ensemble cible-et-bolus est sensiblement la même sur toute la zone à traiter.
Suivant un autre aspect de l'invention, il est proposé un procédé pour déterminer la forme d'un bolus selon la présente invention, ce bolus étant destiné à recevoir une irradiation lors d'une radiothérapie. Le procédé comprend avantageusement les étapes suivantes :
- détermination d'un premier volume définissant une partie profonde de la cible à irradier au sein dudit corps,
- détermination d'un second volume par expansion volumique dudit premier volume, au moins une partie du second volume étant à l'extérieur du corps, ne devrait-on pas expliquer ce qu'est l'expansion volumique (avec une formule idéalement) ?
- détermination de la forme du bolus, cette forme étant en fait la partie du second volume se trouvant à l'extérieur du corps.
L'expansion volumique est une technique connue dans laquelle on met en oeuvre un algorithme tel que défini notamment dans les publications suivantes :
R. Belshi, D. Pontvert, J. C. Rosenwald, and G. Gaboriaud, "Automatic three dimensional expansion of structures applied to determination of Clinical Target Volume in conformal radiotherapy," Int J Rad Oncol Biol Phys, vol. 37, pp. 689-696, 1997; ou
[J. C. Stroom and P. R. M. Storchi, "Automatic calculation of three-dimensional margins around treatment volumes in radiotherapy planning," Phys Med Biol, vol. 42, pp. 745-755, 1997.
Avantageusement, on réalise l'expansion volumique jusqu'à ce que l'épaisseur entre le premier volume et le second volume soit sensiblement constante sur toute la cible.
On peut aussi réaliser l'expansion volumique jusqu'à ce que le contour du second volume soit sensiblement tangent en un point à la surface de la cible, ce point de tangence étant situé dans la zone la plus épaisse de la cible, de façon à ce que le volume étendu englobe toute la cible et inclut même un volume extérieur, celui du bolus en l'occurrence.
Avantageusement, le bolus est élaboré ensuite par moulage, notamment un moulage à froid, à température ambiante.

D'autres avantages et caractéristiques de l'invention apparaîtront à l'examen de la description détaillée d'un mode de mise en oeuvre nullement limitatif, et des dessins annexés, sur lesquels :
La figure 1 est un graphe illustrant le rendement de dose d'un faisceau d'électrons en fonction de la distance de pénétration dans un corps humain, selon l'art antérieur,
La figure 2 est une vue en coupe schématique d'un bolus selon l'art antérieur posé sur un sein devant être irradié pour un traitement en radiothérapie,
La figure 3 est une vue en coupe schématique d'un bolus selon la présente invention plaqué sur un volume lors d'un traitement en radiothérapie,
La figure 4 est un graphe illustrant le rendement de dose d'un faisceau d'électrons en fonction de la distance de pénétration dans un corps humain, en présence (4b) et en absence (4a) d'un bolus selon la présente invention,
Les figures 5 à 7 sont des vues simplifiées illustrant les différentes étapes pour déterminer la forme d'un bolus selon la présente invention, et
La figure 8 est une vue tridimensionnelle d'un bolus obtenu à la suite des étapes décrites sur les figures 5 à 7.

Bien que l'invention n'y soit pas limitée, on va maintenant décrire un bolus utilisé comme compensateur lors d'une radiothérapie par irradiation d'un faisceau d'électrons.

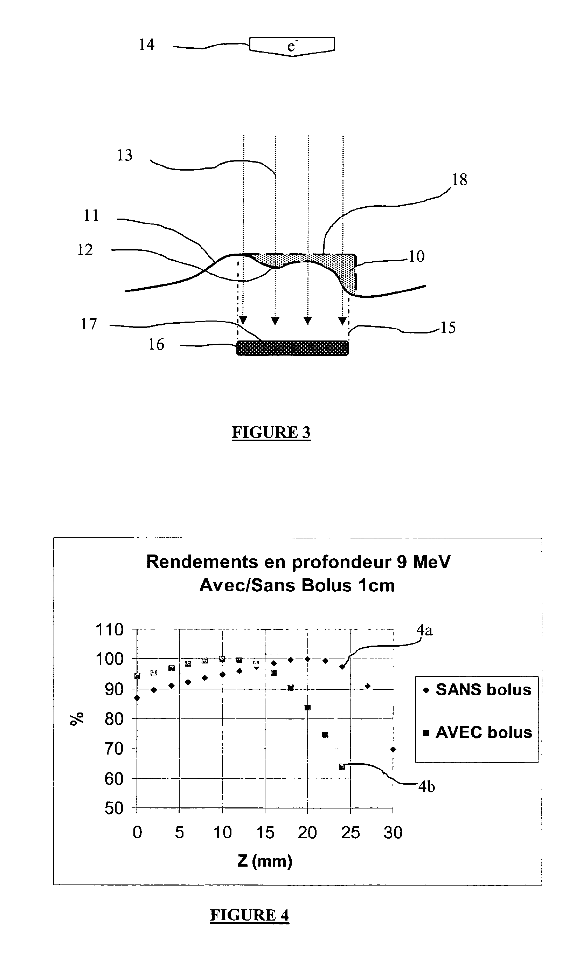

A titre d'exemple, pour traiter le cancer du sein, on peut être amené à réaliser une mastectomie, consistant en un enlèvement chirurgical total d'un sein..
Le bolus selon la présente invention est conçu à partir d'un gel polyuréthane composé d'un mélange de polyol à 90 pourcents et d'isocyanate à 10 pourcents. Il présente une densité de 1. La fluidité de ce gel polyuréthane permet de réaliser un modelage très fin. Sur la figure 3, on voit un volume 11 présentant un creux 12 suite à une opération chirurgicale. La cible 15 est comprise entre la peau (limite du volume 11) et poumon (16). Le traitement consiste en une irradiation de faisceau d'électrons 13 à partir d'une source 14. Les pointillés délimitent la cible 15 qui est le volume du corps soumis au traitement. Avantageusement, le bolus 10 selon la présente invention présente une forme tridimensionnelle telle que les trajets des rayons du faisceau d'électrons 13 traversant le volume de traitement 15 ont sensiblement une même épaisseur. En d'autres termes, la distance entre la surface 18 du bolus 10 et la surface 17 du poumon 16 est sensiblement la même sur tout le volume à traiter. Le bolus 10 a été réalisé à partir du gel polyuréthane puis modelé de façon à épouser parfaitement la surface extérieure du sein 11. Sur la figure 4 on voit un graphe illustrant deux courbes 4a et 4b de rendement en fonction de la profondeur de pénétration du faisceau d'électrons pour une source d'électrons de 9MeV et pour un bolus de 1cm. La courbe 4a correspond à un rendement sans un bolus selon la présente invention. La courbe 4b correspond à un rendement avec un bolus de 1cm d'épaisseur selon la présente invention. Grâce à une densité égale à 1, on voit avantageusement que la courbe 4b a subit une translation de 1 cm par rapport à la courbe 4a, le maximum s'est donc également déplacé de 1cm. Un bolus de 1 cm selon l'invention occasionne un décalage de 1 cm. Ce maximum est atteint pour une profondeur de 10mm, puis la courbe décroît rapidement. Par conséquent, avec le bolus selon la présente invention, on peut concevoir un bolus ayant une forme adaptée pour que le maximum de rendement soit placé au niveau de la cible à irradier, ce qui permet de protéger des organes sains proches de la cible tel que le poumon.

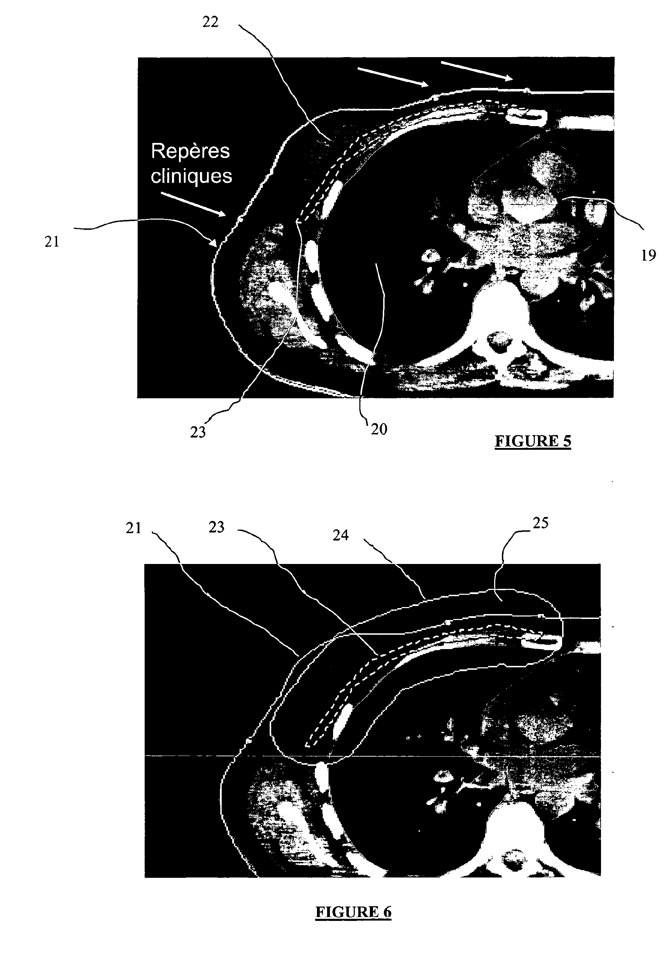

Sur les figures 5 à 7, on voit différentes étapes de fabrication d'un bolus compensateur permettant d'égaliser les épaisseurs traversées entre la surface d'entrée et le bord postérieur du volume cible (paroi costale) à chaque niveau de coupe. La figure 5 est une vue en coupe d'un corps au niveau du thorax. On distingue le coeur 19 et un poumon 20 qui sont des organes à protéger contre le rayonnement d'électrons. On commence par définir la paroi extérieure 21 du thorax 22, en s'aidant notamment de repères cliniques sous forme de billes métalliques sur la peau. On définit ensuite un premier volume 23 qui représente la partie profonde du volume à irradier. Ce premier volume tridimensionnel se trouve dans le thorax. Le bolus doit être réalisé de telle sorte que la distribution de dose entre la peau et le volume 23 soit comprise entre 90 et 100 %.

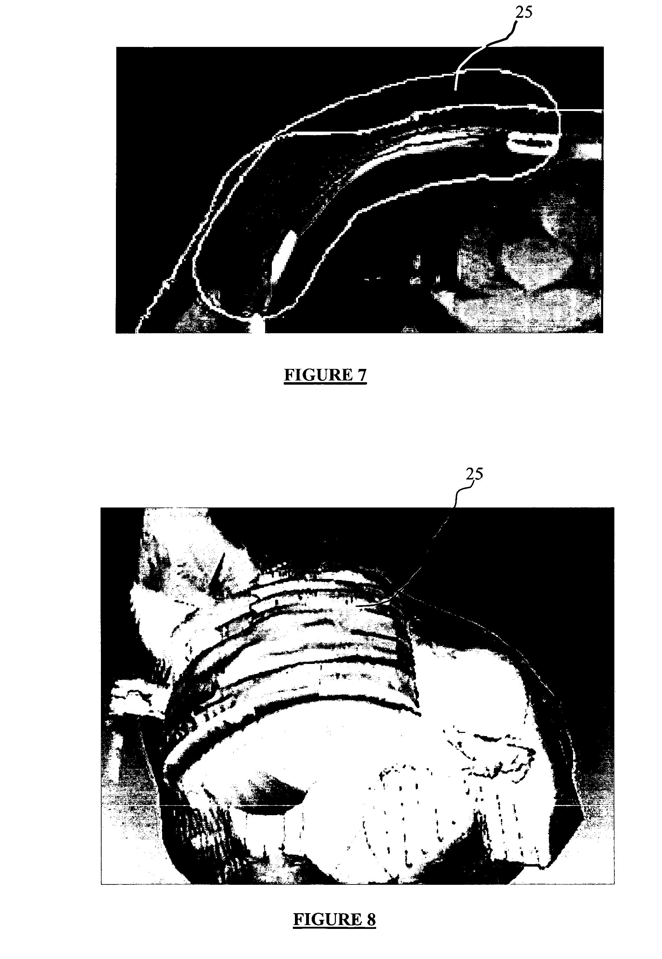

Sur la figure 6, on réalise une expansion à partir du premier volume 23 de façon à générer un second volume 24. Par exemple, on réalise l'expansion jusqu'à obtention d'une surface lisse sur la coupe d'épaisseur maximale. Cette expansion peut avantageusement être réalisée au moyen d'un matériel informatique. Une fois l'expansion terminée, on détermine un troisième volume 25 qui est extérieur au thorax 22 et délimité par le contour du second volume 24 et le contour du thorax 21. Ce troisième volume 25 représente les dimensions du bolus à concevoir. Sur la figure 7 on réalise une délimitation précise du bolus et la figure 8 est une représentation tridimensionnelle du bolus sur le thorax à traiter.

Bien sûr, l'invention n'est pas limitée aux exemples qui viennent d'être décrits et de nombreux aménagements peuvent être apportés à ces exemples sans sortir du cadre de l'invention. En effet, la présente invention peut s'appliquer pour une irradiation autre que celle d'un faisceau d'électrons tel que par exemple le rayonnement gamma ou le rayonnement X. On peut par ailleurs prévoir la réalisation d'un bolus en volume puis un découpage très fin pour conformer le bolus en fonction de la cible.

## Revendications

1. Bolus destiné à recevoir une irradiation lors d'une radiothérapie, **caractérisé en ce qu'**il comprend du gel polyuréthane.

2. Bolus selon la revendication 1, **caractérisé en ce que** le gel polyuréthane présente une densité sensiblement égale à 1.

3. Bolus selon la revendication 1 ou 2, **caractérisé en ce qu'**il est constitué d'un gel polyuréthane composé d'un mélange de polyol et d'isocyanate.

4. Bolus selon la revendication 3, **caractérisé en ce que** le gel polyuréthane comprend au plus quinze pourcents d'isocyanate.

5. Bolus selon la revendication 3, **caractérisé en ce que** le gel polyuréthane comprend sensiblement de huit à dix pourcents d'isocyanate et quatre-vingt-douze à quatre-vingt-dix pourcents de polyol.

6. Bolus selon l'une quelconque des revendications précédentes, **caractérisé en ce qu'**il est destiné à être plaqué sur un corps, et **en ce qu'**il présente une forme permettant de placer sensiblement le maximum de dose d'irradiation dans une cible prédéfinie au sein dudit corps.

7. Bolus selon la revendication 6, **caractérisé en ce que** la forme du bolus est telle que le bolus épouse parfaitement la surface dudit corps et l'épaisseur de l'ensemble cible et bolus est sensiblement la même sur toute la zone à traiter.

8. Procédé pour déterminer la forme d'un bolus selon l'une quelconque des revendications précédentes, ce bolus étant destiné à recevoir une irradiation lors d'une radiothérapie, **caractérisé en ce que** le procédé comprend les étapes suivantes :
- détermination d'un premier volume définissant une partie d'une cible à irradier au sein dudit corps,
- détermination d'un second volume par expansion volumique dudit premier volume ; au moins une partie du second volume étant à l'extérieur du corps,
- détermination de la forme du bolus comme étant ladite partie du second volume se trouvant à l'extérieur du corps.

9. Procédé selon la revendication 8, **caractérisé en ce qu'**on réalise l'expansion volumique jusqu'à ce que l'épaisseur entre le premier volume et le second volume soit sensiblement constante sur toute la cible.

10. Procédé selon la revendication 8, **caractérisé en ce qu'**on réalise l'expansion volumique jusqu'à ce que le contour du second volume soit sensiblement tangent en un point à la surface de la cible, ce point de tangence étant situé dans la zone la plus épaisse de la cible.

11. Procédé selon l'une quelconque des revendications 8 à 10, **caractérisé en ce qu'**on élabore le bolus par moulage.

12. Procédé selon la revendication 11, **caractérisé en ce qu'**on réalise le moulage à froid.
